# EUROPEAN PATENT APPLICATION

(11) **EP 1 932 464 A1**
(43) Date of publication of application: **18.06.2008**
(21) Application number: 06797576.3
(22) Date of filing: 31.08.2006
(51) Int. Cl.: A61B 1/06, A61B 1/04, G02B 23/24

(54) **ELECTRONIC ENDOSCOPE SYSTEM**

(30) Priority: 03.10.2005 JP 2005290047
(71) Applicant: Olympus Corporation, Tokyo 151-0072 (JP)
(72) Inventor: SHIMIZU, Hatsuo, Hachioji-shi Tokyo 192-0024 (JP); NAKAMURA, Mikio, Nishitama-gun Tokyo 190-0182 (JP)
(74) Representative: Winter, Brandl, Fürniss, Hübner Röss, Kaiser, Polte Partnerschaft Patent- und Rechtsanwaltskanzlei
(86) International application number: PCT/JP2006/317700
(87) International publication number: WO 2007/043257

(57) **Abstract**

An electronic endoscope system includes a scope section(100a), an in vitro apparatus(200) which is installed outside a body, and a connecting cord section(100b) which connects the scope section(100a) and the in vitro apparatus(200). Each of the in vitro apparatus(200) and the connecting cord section(100b) includes pads (109, 110, 201, 214) which are structured to be air tight and water tight, and connecting sections(151, 250) for bringing close upon facing each of the pads. Furthermore, for performing a communication of a signal between a pair of pads facing each other, the electronic endoscope system includes a first modulating unit (106) which applies a voltage on one of the pads upon modulating the signal, and a first demodulating unit(202) which demodulates the signal from a change in an electric potential of the other pad.

## Description

### TECHNICAL FIELD

The present invention relates to an electronic endoscope system which communicates in vivo information and an electric power between an apparatus which is introduced inside a body (hereinafter, "in vivo apparatus") and an apparatus which is disposed outside the body (hereinafter, "in vitro apparatus").

### BACKGROUND ART

In a field of a living body, especially a living body of human (hereinafter, "body"), to be examined, particularly in vivo examination, and treatment, in vivo information is communicated outside the body by using an electronic endoscope. Moreover, an electric power etc. has been transmitted to the electronic endoscope (scope section) from an apparatus disposed outside the body, such as a power supply unit. In an electronic endoscope system of a type to be inserted into a human body, a structure which includes an inserting section and an operating section which operates the inserting section is called appropriately as the "scope section". Moreover, a structure which includes the "scope section" and a universal code is called appropriately as "electronic endoscope". Furthermore, a structure which includes the "electronic endoscope" and an apparatus which is disposed outside the body, such as the power supply unit and a video processor unit etc. is called appropriately as "electronic endoscope system".

For example, a structure in which the electric power is supplied from the in vitro apparatus to the scope section by an electromagnetic induction by a coil provided between the in vitro apparatus and the scope section has been proposed (refer to Japanese Patent Application Laid-open Publication No. 2004-159833 for example). In the structure disclosed in the Japanese Patent Application Laid-open Publication No. 2004-159833, a transmission of an image signal and a control signal between the in vitro apparatus and the scope section is performed by demodulating upon transmitting by a wired means or a wireless means (electric waves) after modulating the image signal and the control signal to a wireless frequency.

Moreover, a structure in which a signal transmission between the scope section and the in vitro apparatus is performed by an optical interface has also been proposed (refer to Japanese Patent No. 3615890 for example). In Japanese Patent No. 3615890, furthermore, the electric power is supplied from the in vitro apparatus to the scope section by an electromagnetic coupling (electromagnetic induction).

Moreover, a structure in which an output from a sensor unit at a front end of the inserting section of the scope section is transmitted to an outside by an RFID (Radio Frequency Identification) has been proposed (refer to Japanese Patent Application Laid-open Publication No. 2002-336192).

The electronic endoscope system reuses the scope section inserted into the body, for the other patient. Therefore, it becomes necessary to prevent an infection among the patients via the electronic endoscope or a device for treatment. Consequently, after the examination and treatment is completed, the electronic endoscope, particularly the scope section is washed and disinfected.

For washing (cleaning) and disinfecting the electronic endoscope, a gas for washing and a chemical (liquid) are used. Therefore, it is desirable that the electronic endoscope has an air tight and a water tight (waterproof) structure.

Furthermore, for washing and disinfecting the electronic endoscope easily and efficiently, it is desirable that a connecting section between the electronic endoscope and the in vitro apparatus, and a connecting section between the scope section and the universal code, has to be small sized, and having a shape which eases the washing.

In the structure for transmission disclosed in Japanese Patent Application Laid-open Publication No. 2004-159833, in which the electric power is supplied by the electromagnetic induction, and the image signal and the control signal are transceived (transmitted and received) by the electric wave, it is possible to prevent a poor contact and a damage of connecting pins by minimizing the number of contacts.

However, due to the coil for supplying the electric power by the electromagnetic induction, a size of a contact section is increased. Therefore, the washing of the electronic endoscope is not easy. Furthermore, the coil is not preferable as the coil is a cause of generation of an electromagnetic noise. Thus, due to an antenna for transceiving (transmitting and receiving) the image signal and the control signal by the electric waves, the scope section becomes a large scale, and it becomes difficult to have a structure which facilitates washing. Furthermore, in addition to being weak against a noise generated by a medical equipment such as a radio (electric) knife, there have been constraints such as regulations due to a radio law, and restrictions for preventing an adverse effect on other medical equipments in a hospital.

Moreover, in a structure which has been disclosed in the Japanese Patent No. 3615890, in which the electric power is supplied by the electromagnetic induction, and the signal is transmitted by the optical interface, it is possible to achieve an air tight structure for facilitating the washing.

However, the coil for supplying the electric power by the electromagnetic induction becomes necessary. Therefore, a size of the connecting section between the scope section and the in vitro apparatus becomes big. As a result of this, it is difficult to achieve a structure which facilitates the washing. Moreover, in the signal transmission by the optical interface, in addition to the disinfection and sterilization which are the primary objects of washing, it is necessary to wash an optical interface section such that there is no optical loss. For this reason also, it is difficult to achieve a structure which facilitates the washing.

Moreover, in a structure disclosed in Japanese Patent Application Laid-open Publication No. 2002-336192, in which an output of the sensor unit at the front end of the inserting section of the scope section, such as a temperature sensor and a pressure sensor is transmitted to the outside by the RFID, it is possible to achieve the air tight structure of the sensor unit for facilitating the washing of the sensor unit.

However, in Japanese Patent Application Laid-open Publication No. 2002-336192, there is no description at all about a structure for transmitting image information from the scope section to the in vitro apparatus, and a structure for supplying the electric power supply from the in vitro apparatus to the scope section.

The present invention is made in view of the abovementioned problems, and it is an object of the present invention to provide an electronic endoscope system in which it is not necessary to make the electronic endoscope (scope section) and the in vitro apparatus a large scale by installing the coil for the electromagnetic induction or the antenna for transceiving the electric waves, and which is small in size, and facilitates efficient washing.

### DISCLOSURE OF THE INVENTION

For solving the abovementioned issues, and achieving the object, according to the present invention, it is possible to provide an electronic endoscope system, which includes
an in vivo apparatus, at least a part of which is inserted inside the body,
an in vitro apparatus which is disposed outside the body, and
a connecting cord section which connects the in vivo apparatus and the in vitro apparatus.

At least one of the in vivo apparatus and the in vitro apparatus, and the connecting cord section include a pad which is structured to be air tight and water tight, and a connecting section for bringing near upon facing each of the pads, and further includes
a modulating means which applies a voltage upon modulating a signal on one of the pads, for performing a communication of a signal between the pair of pads facing each other, and a demodulating means which demodulates a signal from a change in an electric potential of the other pad.

Moreover, according to a preferable aspect of the present invention, it is desirable that the pad and the connecting section are provided on the connecting cord section and the in vitro apparatus respectively.

Furthermore, according to another preferable aspect of the present invention, it is desirable that the pad and the connecting section are provided on the connecting cord section and the in vivo apparatus respectively.

According to still another preferable aspect of the present invention, it is desirable that the pad and the connecting section are provided on the connecting cord section and the in vivo apparatus, and the connecting cord section and the in vitro apparatus respectively.

Moreover, according to still another aspect of the present invention, it is desirable that at least the signal which is communicated by a pair of the pads is a signal for transmitting an electric power.

Furthermore, according to still another aspect of the present invention, it is desirable that at least the signal which is communicated by a pair of the pads is an image signal.

### BRIEF DESCRIPTION OF THE DRAWINGS

Fig. 1 is a diagram showing an entire structure of an electronic endoscope system according to a first embodiment of the present invention;
Fig. 2 is a functional block diagram of an electronic endoscope of the first embodiment;
Fig. 3 is a functional block diagram of an in vitro apparatus of the first embodiment;
Fig. 4 is a diagram showing a cross-sectional structure of an area near a connector of the first embodiment;
Fig. 5 is a front structural view of a pad of the first embodiment;
Fig. 6 is a flowchart showing a flow of a signal in the first embodiment;
Fig. 7 is another flowchart showing a flow of the signal in the first embodiment;
Fig. 8 is a diagram showing an entire structure of an electronic endoscope system according to a second embodiment of the present invention;
Fig. 9 is a diagram showing a cross-sectional structure of an area around a connector of the second embodiment; and
Fig. 10 is a diagram showing an entire structure of an electronic endoscope system according to a third embodiment of the present invention.

### BEST MODE FOR CARRYING OUT THE INVENTION

Embodiments of an electronic endoscope system according to the present invention will be described in detail with reference to the accompanying diagrams. However, the present invention is not restricted to the embodiment described below.

### [First embodiment]

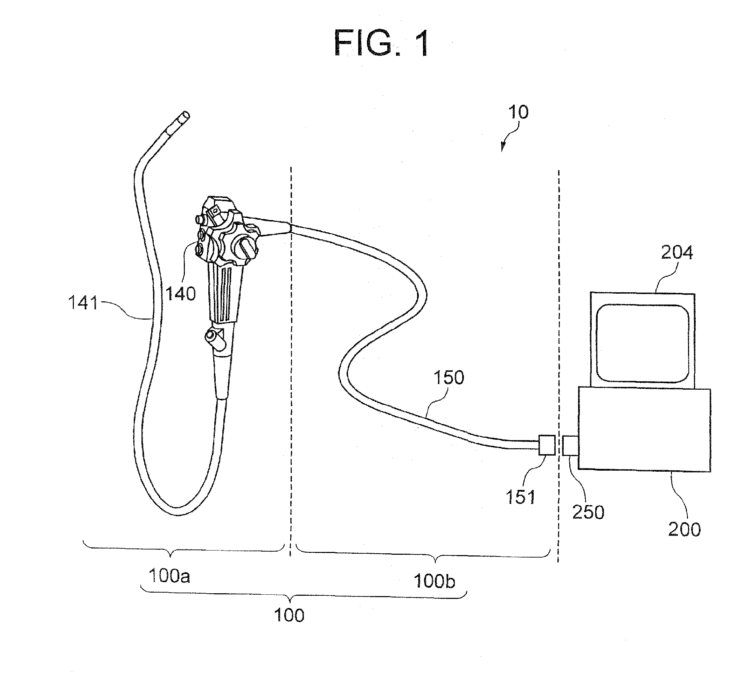

Fig. 1 is a diagram showing a schematic structure of an electronic endoscope system 10 according to a first embodiment of the present invention. The electronic endoscope system 10 includes an electronic endoscope 100 and an in vitro apparatus 200. The electronic endoscope 100 includes a scope section 100a and a connecting cord section 100b. Moreover, the in vitro apparatus 200 includes a power supply unit, a video processor (not shown in the diagram) which performs processing of an image signal from the electronic endoscope 100, and a display unit 204 which performs a monitor display of the image signal from the video processor. The scope section 100a corresponds to the in vivo apparatus.

The scope section 100a is mainly divided into an operating section 140 and an inserting section 141. The inserting section 141 includes a long and slender member having a flexibility, which can be inserted into a body cavity of a patient. A user (not shown in the diagram) can perform various operations by an angle knob provided on the operating section 140.

Moreover, the connecting cord section 100b is extended from the operating section 140. The connecting cord section 100b includes a universal cord 150 and a connector 151. An end portion of the connecting cord section 100b on a side of the scope section 100a is formed integrally with the operating section 140. Whereas, the connector 151 is formed at an end portion of the connecting cord section 100b, on a side of the in vitro apparatus 200.

The universal cord 150 is connected to the in vitro apparatus via the connector 151 and a connector 250. Details of the connectors 151 and 250 will be described later.

Moreover, the universal cord 150 communicates signals such as a CCD driving signal and a power supply voltage signal from the video processor and the power supply unit to the scope section 100a, and communicates an image signal from the scope section 100a to the video processor. Peripheral equipments such as a VTR (video tape recorder) deck and a video printer which are not shown in the diagram can be connected to the video processor inside the in vitro apparatus 200. The video processor can perform a predetermined signal processing on the image signal from the scope section 100a, and can display an endoscope image on a display screen of the display unit 204.

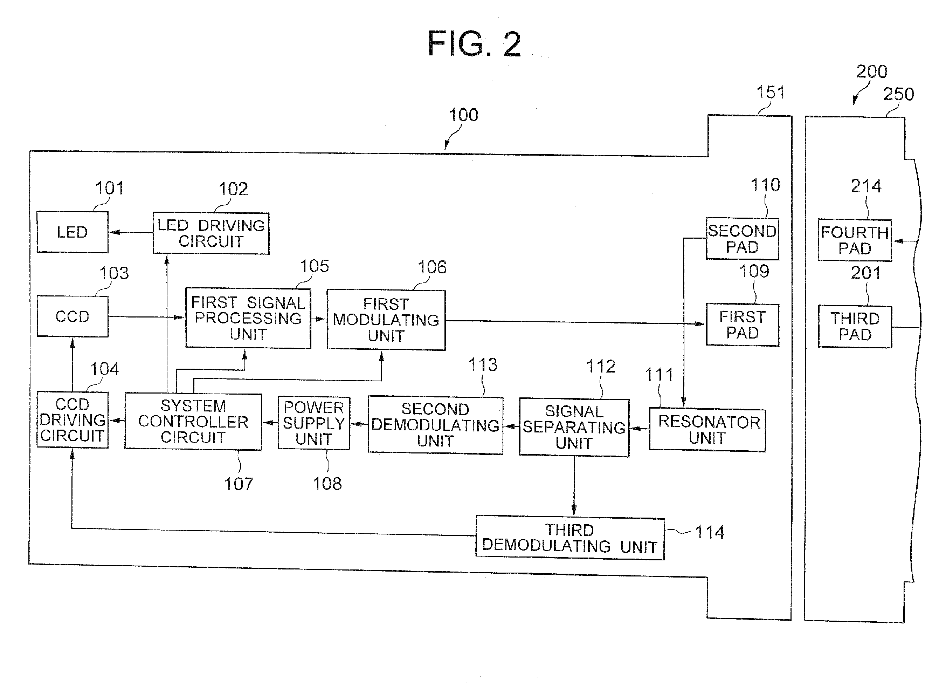

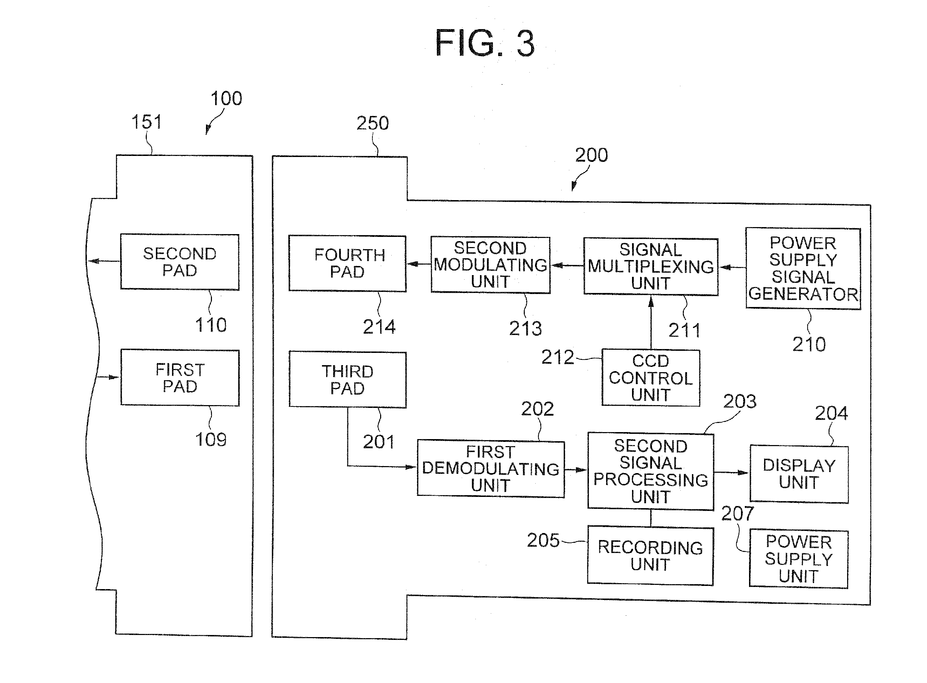

Fig. 2 is a functional block diagram of the electronic endoscope 100. Moreover, Fig. 3 is a functional block diagram of the in vitro apparatus 200. In the first embodiment, a bidirectional signal communication between the electronic endoscope 100 and the in vitro apparatus 200 is possible. First of all, the signal communication from the electronic endoscope 100 to the in vitro apparatus 200 will be described below. Next, the signal communication from the in vitro apparatus 200 to the electronic endoscope 100 will be described.

The electronic endoscope 100 includes an LED (light emitting diode) 101 for illuminating an imaging area at the time of taking a picture of inside of the body, an LED driving circuit 102 which controls a driving of the LED 101, and a CCD (charge coupled device) 103 which performs imaging of the area inside the body illuminated by the LED 101. Moreover, the electronic endoscope 100 includes a CCD driving circuit 104, a first signal processing unit 105, a first modulating unit 106, a first pad 109, and a system control circuit 107. The CCD driving circuit 104 controls a driving of the CCD 103. The first signal processing unit 105 performs a processing of an image data (image signal) imaged by the CCD 103. The first modulating unit 106 modulates an in vivo information signal from the first signal processing unit 105. A modulated voltage from the first modulating unit 106 is applied on the first pad 109. The system control circuit 107 controls an operation of the LED driving circuit 102, the CCD driving circuit 104, the first signal processing unit 105, and the first modulating unit 106. Moreover, a power supply unit 108 supplies an electric power to each unit and each circuit in the electronic endoscope 100, according to a power supply voltage signal from an in vitro apparatus 200 which will be described later.

The CCD 103 acquires in vivo information such as in vivo image information. The CCD 103 corresponds to an imaging section and has a function as an in vivo information sensor. Apart from the CCD 103, a CMOS (complementary metal oxide semiconductor) can be used as the imaging section. A window formed by a transparent material is provided near the imaging section. The imaging section captures in vivo images through the window.

The CCD 103 is connected to the CCD driving circuit 104. The CCD driving circuit 104 outputs to the CCD 103 an operation signal for acquiring the in vivo information. The CCD 103 is connected to the first signal processing unit 105. The first signal processing unit 105 has a function as an in vivo information processing unit. The first signal processing unit 105 includes circuits such as a data compression circuit and an image converting circuit for output from the CCD 103, for example. Moreover, the first signal processing unit 105 generates an in vivo information signal from an output signal of the CCD 103, and outputs the in vivo information signal which is generated.

The CCD driving circuit 104 and the first signal processing unit 105 are connected to the first modulating unit 106 via the system control circuit 107. The first modulating unit 106 modulates the output signal from the first signal processing unit 105, and applies a voltage to the first pad 109. When the modulation is a general type of modulation such as an amplitude modulation, a frequency modulation, and a phase modulation, any type of such modulation can be used. Here, the first pad 109 is structured to be air tight and water tight.

Next, the in vitro apparatus 200 will be described. The in vitro apparatus 200 includes a first demodulating unit 202, a second signal processing unit 203, a recording unit 205, and a power supply unit 207. The first demodulating unit 202 demodulates the output signal of the first signal processing unit 105, from a change in an electric potential of a surface of a third pad 201.

By applying to the first pad 109 a voltage in which the output signal from the first signal processing unit 105 is modulated, there occurs to be a change in the electric potential on the surface of the third pad 201. The first demodulating unit 202 demodulates the output signal from the first signal processing unit 105. Accordingly, it is possible to realize a communication from a side of the electronic endoscope 100 to the side of the in vitro apparatus 200.

The first demodulating unit 202 is connected to the second signal processing unit 203. The second signal processing unit 203 is a circuit such as a decompression circuit for compressed data, and a correction / enhancing circuit of the image information. The second signal processing unit 203 performs a signal processing for acquiring the required in vivo information such as image information, based on the output signal from the first signal processing unit 105 which is demodulated by the first demodulating unit 202.

Moreover, the second signal processing unit 203 is connected to the display unit 204. The display unit 204 is a monitor such as a liquid crystal display. The display unit 204 displays the in vivo information which is processed in the second signal processing unit 203. In Fig. 1, the display unit 204 is not provided on the in vitro apparatus 200 but provided elsewhere. However, without restricting to the structure in which the display unit 204 is not provided on the in vitro apparatus 200, the structure may be such that the display unit 204 is provided on the in vitro apparatus 200.

The recording unit 205 is connected to the first demodulating unit 202 or the second signal processing unit 203. The recording unit 205 includes a memory such as a semiconductor memory. The recording unit 205 records and stores the output signal from the first signal processing unit 105 which is demodulated by the first demodulating unit 202, or the in vivo information which is processed by the second signal processing unit 203.

Moreover, the power supply unit 207 supplies the electric power to the first demodulating unit 202, the second signal processing unit 203, and the recording unit 205.

According to the first embodiment, the electronic endoscope 100 and the in vitro apparatus 200 can communicate the in vivo information to the outside of the body independent of electric waves and electric current. Inventors of the present invention have been considering that the information can be communicated by electrostatic induction. The inventors made a practical apparatus, and tested and confirmed practically that such a communication is possible.

Thus, in the first embodiment, a size of the electronic endoscope 100 and the in vitro apparatus 200 is not required to be increased by installing an antenna and a transmitting circuit respectively. Therefore, it is possible to provide a small size electronic endoscope system which enables to reduce a strain on the patient.

Furthermore, in the first embodiment, the first pad 109 which is formed on the side of the electronic endoscope 100 and the third pad 201 which is formed on the side of the in vitro apparatus 200 are disposed at positions facing each other so as to be coupled electrostatically. Similarly, a second pad 110 formed on the side of the electronic endoscope 100, and a fourth pad 214 which is formed on the side of the in vitro apparatus 200, which will be described later, are disposed at positions facing each other so as to be coupled electrostatically.

Moreover, the connector 151 and the connector 250 are structured to be detachable by a mechanism such as screw-joining mechanism using a screw, a detachable mechanism using a magnet, and a latch mechanism. The connectors 151 and 250 correspond to the connecting section.

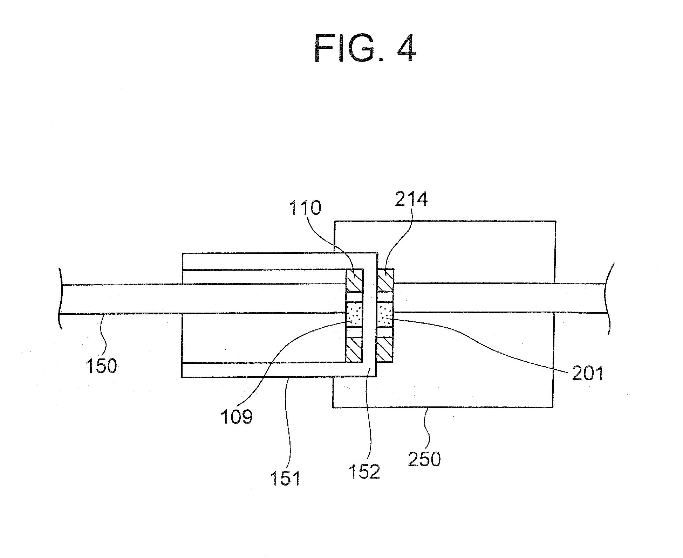

Moreover, in the first embodiment, as shown in Fig. 4, each of the first pad 109 and the second pad 110 have a structure in which a surface of an electroconductive material in a form of a plate is covered by an insulating material 152. The electronic endoscope 100 including the connector 151 has a sealed structure. A thickness of the insulating material 152 is about 1 mm or less.

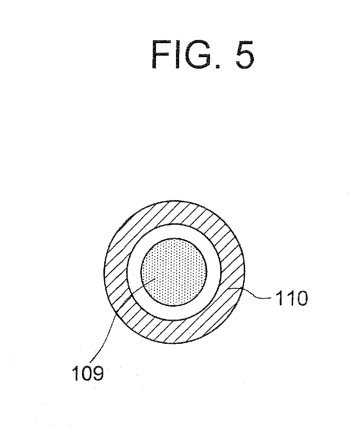

Fig. 5 shows a structure of the connector 151 as viewed from a front side. As shown in Fig. 5, an electroconductive material forming the second pad 110 having a shape of a ring is formed around an outer circumference of the electroconductive material which forms the first pad 109 having a circular shape. However, without restricting to such a structure, it is also possible to adopt another arrangement structure such as a structure in which the first pad 109 and the second pad 110 are disposed by arranging side by side.

Furthermore, it is also possible to make one pad to serve both as the first pad 109 and the second pad 110, in other words to let a structure in which the purpose is served by one electroconductive material, by using different modulation frequency for each of the first modulating unit 106 on the side of the electronic endoscope 100 and a second modulating unit 213 on the side of the in vitro apparatus 200, which will be described later.

Next, a communication of a signal from the in vitro apparatus 200 to the electronic endoscope 100 will be described. In Fig. 3, the in vitro apparatus 200 further includes a power supply signal generator 210, a CCD control unit 212, and a signal multiplexing unit 211. The power supply signal generator 210 outputs a power supply voltage signal of a predetermined frequency. The CCD control unit 212 outputs a control signal to be sent to the CCD 103, such as a control signal of CCD sensitivity.

The signal multiplexing unit 211 outputs upon superimposing the control signal output from the CCD control unit 212 to the CCD 103, on a power supply voltage signal which is output from the power supply signal generator 210. The signal multiplexing unit 211 is connected to the second modulating unit 213. Moreover, the second modulating unit 213 is connected to the fourth pad 214. The second modulating unit 213 modulates an output signal from the signal multiplexing unit 211 and applies the voltage to the fourth pad 214.

Next, the description will be continued upon coming back to Fig. 2. The second pad 110 is connected to a resonator unit 111 which is provided inside the electronic endoscope 100. The resonator unit 111 outputs upon extracting a frequency component which is modulated by the second modulating unit 214 from the change in the electric potential of the second pad 110 due to an electrical resonance.

The resonator unit 111 is connected to a signal separating unit 112. The signal separating unit 112 is connected to a second demodulating unit 113 and a third demodulating unit 114.

The signal separating unit 112 separates the change in the electric potential of the second pad 110 which is output upon extracting by the resonator unit 111, into a power supply voltage signal component and a control signal component to the CCD 103. Moreover, the signal separating unit 112 outputs the power supply voltage signal component to the second demodulating unit 113. Furthermore, the signal separating unit 112 outputs the control signal component to the CCD 103, to the third demodulating unit 114.

The second demodulating unit 113 demodulates a power supply voltage signal output from the power supply signal generator 210, based on the voltage signal component of the change in the electric potential of the second pad 110 which is output from the signal separating unit 112.

The second demodulating unit 113 is connected to the power supply unit 108. The power supply unit 108 supplies power for operating each unit and each circuit in the electronic endoscope 100, from the power supply voltage signal demodulated by the second demodulating unit 113, via the system control circuit 107.

Thus, the voltage in which a signal on which the control signal to the CCD 103 which is output by the CCD control unit 212 is superimposed, is modulated, is applied to the power supply voltage signal which is output to the fourth pad 214 by the power supply signal generator 210. Moreover, on the side of the electronic endoscope 100, the power supply voltage signal output by the power supply signal generator 210 is demodulated upon separating from the change in the electric potential of a surface of the second pad 110 which has occurred due to applying the voltage. Accordingly, it is possible to supply the electric power from the in vitro apparatus 200 to the electronic endoscope 100. As a result, in the electronic endoscope system 10 of the first embodiment, even when compared to a power supply by the electromagnetic induction, the size of the system is not increased due to a winding etc.

Furthermore, the third demodulating unit 114 demodulates the control signal of the CCD 113 which is output by the CCD control unit 212, from the voltage signal component of the change in the electric potential of the second pad 110 which is output by the signal separating unit 112.

The third demodulating unit 114 is connected to the CCD driving circuit 104. The CCD 103 is driven based on the control signal to the CCD 103 from the CCD control unit 212 which is demodulated, such as an instruction signal of sensitivity control.

Thus, the voltage in which the signal on which the control signal to the CCD 103 is output by the CCD control unit 212 is superimposed, is demodulated, is applied to the power supply voltage signal which is output to the fourth pad 214 by the power supply signal generator 210. Moreover, on the side of the electronic endoscope 100, the control signal to the CCD 103 output by the CCD control unit 212 is demodulated upon separating from the change in the electric potential of the surface of the second pad which is occurred due to applying the voltage. Accordingly, it is possible to realize a signal communication from the in vitro apparatus 200 to the electronic endoscope 100. As a result, in the electronic endoscope system 10 of the first embodiment, the size of the system is not increased due to installing an antenna for transceiving (transmitting and/or receiving) the electric waves.

Moreover, it is possible to realize an air tight and a water tight structure necessary for the electronic endoscope 100, by the first pad 109 and the second pad 110. Consequently, in the first embodiment, it is not necessary to provide a water proof cap separately on the connector 151 while washing (or cleaning). Furthermore, the connector 151 is small sized with a simple structure without unevenness. As a result, it is possible to wash easily and efficiently the electronic endoscope 100.

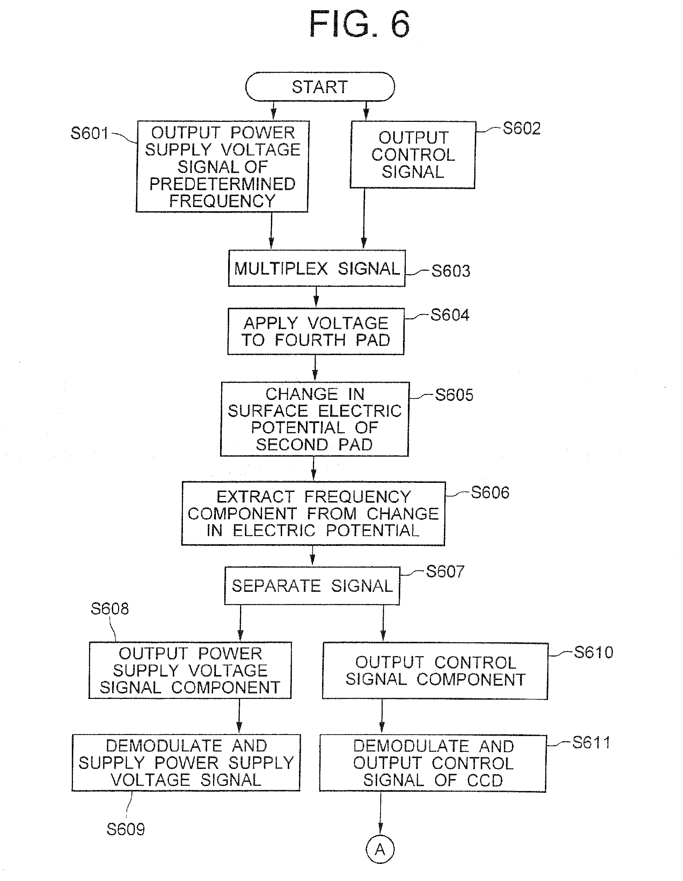

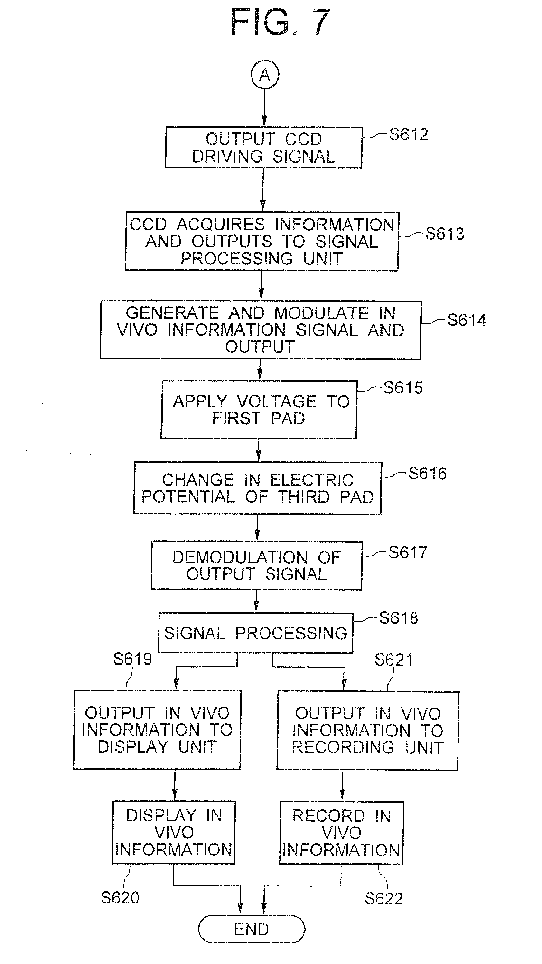

Next, a flow of a signal in the first embodiment described above will be described in further details, with reference to flowcharts. Each of Fig. 6 and Fig. 7 is a flowchart showing the flow of the signal in the second embodiment.

At step S601, the power supply signal generator 210 outputs a power supply voltage signal of a predetermined frequency to the signal multiplexing unit 211. At step S602, the CCD control unit 212 outputs to the signal multiplexing unit 211, a control signal to the CCD 113.

At step S603, the signal multiplexing unit 211 superimposes the control signal to the CCD 103 which is output by the CCD control unit 212, on the power supply voltage signal which is output by the power supply signal generator 210, and outputs to the second modulating unit 213.

At step S604, the second modulating unit 213 modulates the output signal of the signal multiplexing unit 211, and applies voltage to the fourth pad 214.

At step S605, the electric potential of the surface of the second pad 110 is changed due to the voltage applied to the fourth pad 214 which has modulated the output signal of the signal multiplexing unit 211.

At step S606, the resonator unit 111 extracts a frequency component which is output upon modulating by the second modulating unit 213 from the change in the electric potential of the second pad 110 by the electrical resonance, and outputs to the signal separating unit 112.

At step S607, the signal separating unit 112 separates the change in the electric potential of the second pad 110 which is extracted by the resonator unit 111, into a power supply voltage signal component, and a control signal component to the CCD 103.

At step S608, the signal separating unit 112 outputs the power supply voltage signal component separated by the signal separating unit 112, to the second demodulating unit 113.

At step S609, the second demodulating unit 113 demodulates a power supply voltage signal output from the power supply signal generator 210, from the change in the electric potential of the second pad 110. Further, the power supply voltage signal (electric power) which is modulated is supplied to each unit and each circuit etc. in the electronic endoscope 100 via the power supply unit 108.

At step S610, the signal separating unit 112 outputs to the third demodulating unit 114, the control signal component to the CCD 103. At step S611, the third demodulating unit 114 demodulates the control signal to the CCD 103 which is output by the CCD control unit 212, from the change in the electric potential of the second pad 110. Further, the third demodulating unit 114 outputs the control signal which is demodulated, to the CCD driving circuit 104.

Next, at step S612 in Fig. 7, the CCD driving circuit 104 outputs a driving signal to the CCD 103. At step S613, the CCD 103 acquires in vivo information (performs imaging). Further, the CCD 103 outputs the in vivo information which is acquired, to the signal processing unit 105.

At step S614, the first signal processing unit 105 generates an in vivo information signal from the output signal of the CCD 103. Further, the signal processing unit 105 outputs the in vivo information signal generated, to the first modulating unit 106.

At step S615, the first modulating unit 106 modulates the output signal from the signal processing unit 105. Further, the first modulating unit 106 applies voltage to the first pad 109 according to the output signal which is modulated.

At step S616, the electric potential of the surface of the third pad 201 is changed due to the voltage applied to the first pad 109, in which the output signal from the signal processing unit 105 is modulated.

At step S617, the first demodulating unit 202 demodulates the output signal of the signal processing unit 105, based on the change in the electric potential of the surface of the third pad 201. Further, the first demodulating unit 202 outputs the demodulated output signal, to the second signal processing unit 203.

At step S618, the second signal processing unit 203 performs a signal processing for acquiring the required in vivo information, from the output signal of the first signal processing unit 105, which is demodulated by the first demodulating unit 202.

At step S619, the second signal processing unit 203 outputs the in vivo information acquired during the signal processing, to the display unit 204. At step S620, the display unit 204 displays the in vivo information.

At step S621, the second signal processing unit 203 outputs the in vivo information acquired during the signal processing, to the recording unit 205. At step S622, the recording unit 205 records and stores the in vivo information.

### [Second embodiment]

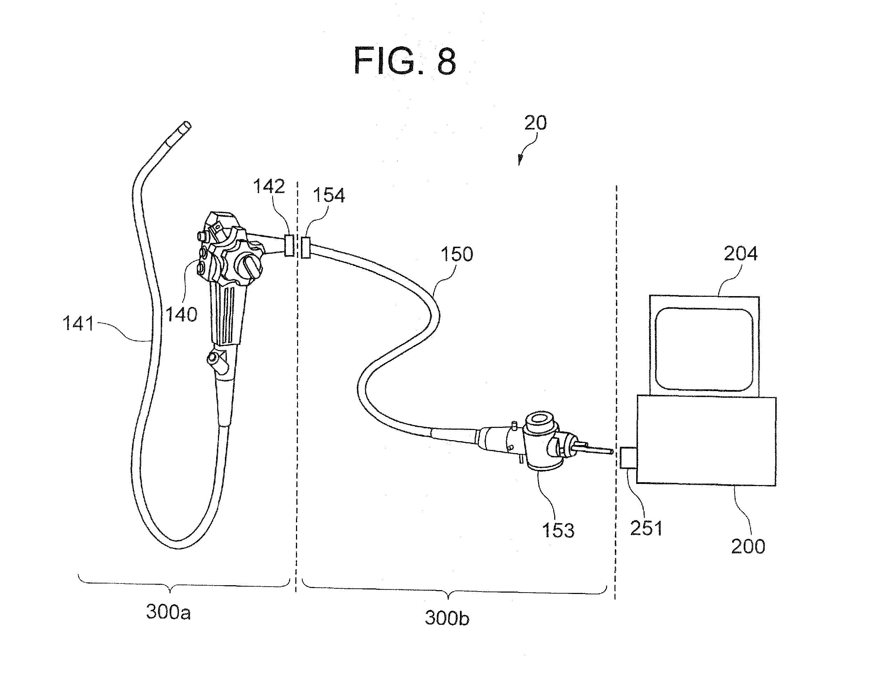

Next, an electronic endoscope system 20 according to a second embodiment of the present invention will be described. Fig. 8 shows a schematic structure of the electronic endoscope system 20. Same reference numerals are used for components which are same as in the first embodiment, and the repeated description is omitted.

In the second embodiment, a connector 142 is formed at a portion of a scope section 300a, which is extended from the operating section 140. Moreover, a connector 154 is formed at an end portion of a connecting cord section 300b on a side of the scope section 300a.

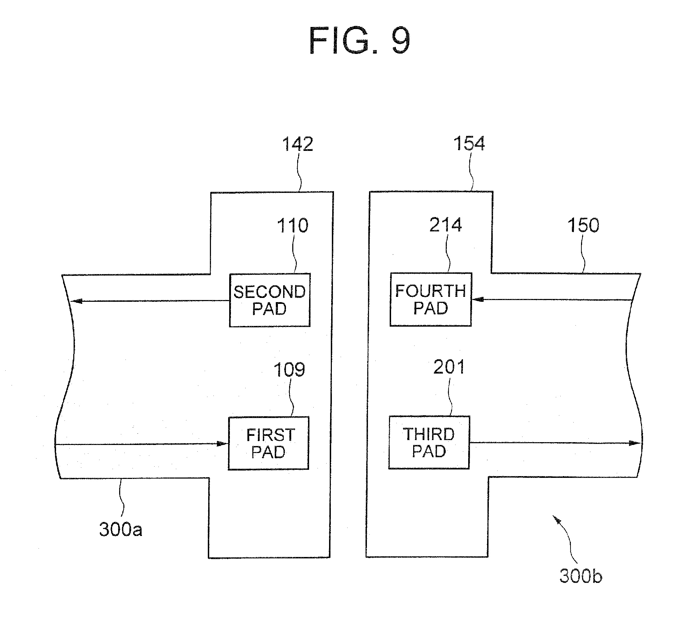

Fig. 9 shows an enlarged structure of an area near the connectors 142 and 154. The structure of the connectors 142 and 154 is same as the structure of the connectors 151 and 250 respectively. Moreover, the first pad 109 and the third pad 201 are disposed adjacent facing each other so as to be coupled electrostatically. Similarly, the second pad 110 and the fourth pad 214 are disposed adjacent facing each other so as to be coupled electrostatically.

A signal which is communicated by the first pad 109 and the third pad 201 is an image signal. Moreover, a signal which is communicated by the second pad 110 and the fourth pad 214 is a power supply voltage signal. The first pad 109, the second pad 110, and the connectors 142 and 154 are structured to be air tight and water tight.

Consequently, in the second embodiment, it is not necessary to provide a water proof cap separately on the connectors 142 and 154 while washing (cleaning). Furthermore, the connectors 142 and 154 are small sized with a simple structure without unevenness. As a result, it is possible to wash easily and efficiently the scope section 300a A connector 153 as in a conventional structure is formed at the other end portion of the connecting cord section 300b. Moreover, another connector 251 of a conventional type for connecting the conventional type connector 153 is provided in the in vitro apparatus 200.

### [Third embodiment]

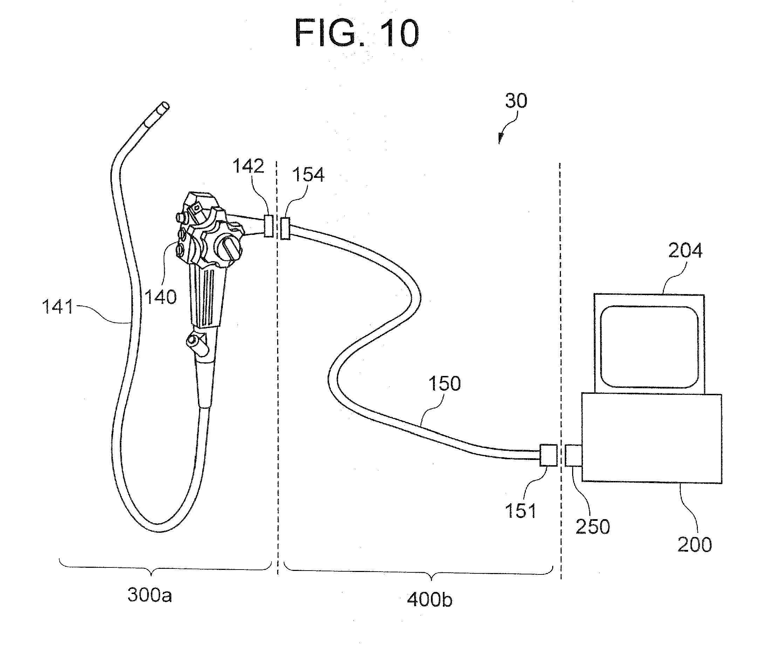

Next, an electronic endoscope system 30 according to a third embodiment of the present invention will be described. Fig. 10 shows a schematic structure of the electronic endoscope system 30. Same reference numerals are used for components which are same as in the first embodiment, and the repeated description is omitted.

In the third embodiment, similarly as in the first embodiment, the connector 151 is formed at the end portion of the universal cable 150. Moreover, the connector 250 is formed on the in vitro apparatus 200.

Furthermore, similarly as in the second embodiment, the connector 142 is formed at the portion of the scope section 300a, which is extended from the operating section 140. Moreover, the connector 154 is formed at the end portion of the connecting cord section 400b on the side of the scope section 300a.

Accordingly, in the third embodiment, it is possible to connect and separate easily each of the scope section 300a, the connecting cord section 400b, and the in vitro apparatus 200. Moreover, a pad provided in each of the connectors 142, 151, 154, and 250 is structured to be air tight and water tight.

Therefore, each of the scope section 300a and the connecting cord section 400b can be washed when separated. As a result, it is possible to wash easily and efficiently the scope section 300a and the connecting cord section 400b separately, according to an amount of sterilization and a level of washing.

The electronic endoscope system in each of the embodiments mentioned above is structured to capture an image of inside of the body, by providing a CCD etc. However, the electronic endoscope is not restricted to such a structure, and may be let to be an apparatus which acquires other in vivo information such as information of temperature and pH of the body.

Moreover, the present invention is not restricted to the electronic endoscope system, and can also be applied to an information terminal such as a PDA, an IC recorder, and a digital camera of waterproof specifications, a charging mechanism and a transmission mechanism for information in various equipments such as a cordless telephone, and a mobile telephone.

Furthermore, the present invention can have various modified embodiments (examples) which fairly fall within the basic teachings herein set forth.

According to an electronic endoscope system according to the present invention, a voltage is applied between a pair of pads, upon modulating a signal in a pad of one of apparatuses, and a connecting cord section. Moreover, in the other apparatus and the connecting cord section, a signal is demodulated from a change in an electric potential of the pad. Accordingly, for example, between the connecting cord section and an in vivo apparatus, and between the connecting cord section and an in vitro apparatus, it is possible to perform a communication of information without using electric waves and electric current. Therefore, for example, when the information is to be communicated from the in vivo apparatus to the in vitro apparatus, the in vivo apparatus is not required to have an antenna and a transmitting circuit, and consequently it is possible to reduce a size of the in vivo apparatus. Moreover, also regarding the in vitro apparatus, a structure in which a plurality of antennas for receiving a signal is disposed near a body, such as a body of a patient, a detection of a weak electric current and a demodulating circuit, are not required. Consequently, the in vivo apparatus and the in vitro apparatus are not required to be large scale by installing the antenna etc. As a result, it is possible to provide an electronic endoscope system having a small size. Moreover, each pad from the pair of pads has an air tight and a water tight structure. Therefore, at the time of washing and disinfecting the in vivo apparatus and the connecting cord section by using a gas and a chemical (liquid) for cleaning, the connecting cord section is not required to be sealed by a water proof cap etc. Furthermore, since the structure is small sized as described above, it is possible to wash and disinfect the in vivo apparatus and the connecting cord section easily and efficiently. Thus, according to the present invention, it is not necessary to make the electronic endoscope (scope section) and the in vitro apparatus of a large scale by a cord for an electromagnetic induction and an antenna for transceiving electric waves, and it is possible to provide an electronic endoscope system having a small size which can be washed efficiently with ease.

### INDUSTRIAL APPLICABILITY

Thus, the electronic endoscope system according to the present invention is appropriate for an electronic endoscope system which is small sized and can be washed easily.

## Claims

1. An electronic endoscope system, comprising:
an in vivo apparatus, at least a part of which is inserted inside a body;
an in vitro apparatus which is disposed outside the body; and
a connecting cord section which connects the in vivo apparatus and the in vitro apparatus, wherein
at least one of the in vivo apparatus and the in vitro apparatus, and the connecting cord section include a pad which is structured to be air tight and water tight, and a connecting section for bringing near upon facing each of the pads, and further comprising:
a modulating unit which applies a voltage on one of the pads upon modulating a signal, for performing a communication of a signal between the pair of pads facing each other; and
a demodulating unit which demodulates a signal from a change in an electric potential of the other pad, for performing a communication of a signal between the pair of pads facing each other.

2. The electronic endoscope system according to claim 1, wherein
the pad and the connecting section are provided on the connecting cord section and the in vitro apparatus respectively.

3. The electronic endoscope system according to claim 2, wherein
at least the signal which is communicated by a pair of the pads is a signal for transmitting an electric power.

4. The electronic endoscope system according to claim 2, wherein
at least the signal which is communicated by a pair of the pads is an image signal.

5. The electronic endoscope system according to claim 1, wherein
the pad and the connecting section are provided on the connecting cord section and the in vivo apparatus respectively.

6. The electronic endoscope system according to claim 5, wherein
at least the signal which is communicated by a pair of the pads is a signal for transmitting an electric power.

7. The electronic endoscope system according to claim 5, wherein
at least the signal which is communicated by a pair of the pads is an image signal.

8. The electronic endoscope system according to claim 1, wherein
the pad and the connecting section are provided on the connecting cord section and the in vivo apparatus, and the connecting cord section and the in vitro apparatus respectively.

9. The electronic endoscope system according to claim 8, wherein
at least the signal which is communicated by a pair of the pads is a signal for transmitting an electric power.

10. The electronic endoscope system according to claim 8, wherein
at least the signal which is communicated by a pair of the pads is an image signal.

11. The electronic endoscope system according to claim 1, wherein
at least the signal which is communicated by a pair of the pads is a signal for transmitting an electric power.

12. The electronic endoscope system according to claim 1, wherein
at least the signal which is communicated by a pair of the pads is an image signal.
